# EUROPEAN PATENT SPECIFICATION

(11) **EP 4 509 116 B1**
(45) Date of publication and mention of the grant of the patent: **26.08.2026**
(21) Application number: 23763381.3
(22) Date of filing: 27.02.2023
(51) Int. Cl.: A61K 9/06, A61L 26/00, A61K 47/36, C08B 37/08, A61L 15/60

(54) **METHOD FOR PREPARING MEDICAL HYDROGEL AND MEDICAL HYDROGEL**
VERFAHREN ZUR HERSTELLUNG EINES MEDIZINISCHEN HYDROGELS UND MEDIZINISCHES HYDROGEL
PROCÉDÉ DE PRÉPARATION D'UN HYDROGEL MÉDICAL ET HYDROGEL MÉDICAL

(30) Priority: 01.03.2022 JP 2022030659
(43) Date of publication of application: 19.02.2025
(73) Proprietor: Kagoshima University, Kagoshima-shi, Kagoshima 890-8580 (JP)
(72) Inventor: TAKEI Takayuki, Kagoshima-shi, Kagoshima 890-8580 (JP); YOSHIDA Masahiro, Kagoshima-shi, Kagoshima 890-8580 (JP); YAMASHITA Yusuke, Kagoshima-shi, Kagoshima 890-8580 (JP)
(74) Representative: v. Bezold & Partner Patentanwälte - PartG mbB
(86) International application number: PCT/JP2023/006946
(87) International publication number: WO 2023/167123

(56) References cited:
- WO-A1-2012/105685
- TAKAYUKI TAKEI, SO DANJO, SHOGO SAKOGUCHI, SADAO TANAKA, TAKUMA YOSHINAGA, HIROTO NISHIMATA, MASAHIRO YOSHIDA: "Autoclavable physically-crosslinked chitosan cryogel as a wound dressing", JOURNAL OF BIOSCIENCE AND BIOENGINEERING, ELSEVIER, AMSTERDAM, NL, vol. 125, no. 4, 1 April 2018 (2018-04-01), NL , pages 490 - 495, XP055686809, ISSN: 1389-1723, DOI: 10.1016/j.jbiosc.2017.10.015

## Description

### Technical Field

The present disclosure relates to a method of preparing a medical hydrogel.

### Background Art

Wound dressings are required to have a property that promotes wound healing. Hydrogel-like wound dressings capable of providing a moist environment to a wound site have been reported to promote formation of a granulation tissue and re-epithelialization at the wound site, thereby promoting healing.

Chitosan is a natural polysaccharide that shows antibacterial action and wound-healing effect. Because of its properties, chitosan has been widely used as a wound dressing material. However, since chitosan is poorly soluble in water at physiological pHs, preparation of a hydrogel-like chitosan wound dressing is difficult. On the other hand, although a chitosan derivative that is water-soluble at a physiological pH has been reported, preparation of a hydrogel from the chitosan derivative requires use of a cross-linking agent such as glutaraldehyde. In this case, biological toxicity of the cross-linking agent remaining in the gel may be problematic.

In view of such circumstances, a method of preparing a chitosan-derived hydrogel without using additives harmful to the living body has been proposed (Patent Literature 1). In Patent Literature 1, a hydrogel having no biological toxicity was prepared by freezing and thawing of an aqueous solution containing an aldonic acid-modified chitosan. Patent Literature 2 discloses a method of preparing a chitosan-derived hydrogel without use of harmful additives, wherein an aqueous solution of a chitosan-gluconic acid conjugate is placed in a mold and subjected to freeze-thawing to obtain a physically crosslinked cryogel. The resulting cryogels are described as useful for wound dressing applications.

### Citation List

### Patent Literature

Patent Literature 1: International Publication No. WO 2012/105685;
Patent Literature 2: Takayuki Takei, So Danjo, Shogo Sakoguchi, Sadao Tanaka, Takuma Yoshinaga, Hiroto Nishimata, Masahiro Yoshida: "Autoclavable physicallycrosslinked chitosan cryogel as a wound dressing", Journal of bioscience and bioengineering, ELSEVIER, AMSTERDAM, NL, vol. 125, no. 4, 1 April 2018 (2018-04-01), pages 490-495.

### Summary of Invention

### Technical Problem

In Patent Literature 1, when the hydrogel is used for medical applications such as wound dressings, sterilization is required after the preparation of the hydrogel, which means that the process requires the steps of freezing, thawing, and sterilization.

The present disclosure was made in view of the above problem, and an objective of the disclosure is to provide a method of preparing a medical hydrogel whose sterilization and gelling can be achieved by a simple method.

### Solution to Problem

The method of preparing a medical hydrogel according to the present disclosure is
a method of preparing a medical hydrogel derived from a chitosan derivative containing aldonic acid bound by dehydration condensation to an amino group of a glucosamine unit of chitosan,
the method including placing an aqueous solution containing the chitosan derivative in a mold having a predetermined shape, and then subjecting the aqueous solution to high-pressure steam sterilization, to obtain a sterile medical hydrogel having the predetermined shape.

The high-pressure steam sterilization is preferably carried out at not less than 121°C for 15 minutes to 2 hours.

Preferably, the chitosan has a deacetylation rate of 70 to 90%, and the chitosan derivative has an introduction rate of the aldonic acid of 5 to 60%.

The aldonic acid is preferably gluconic acid.

The resulting medical hydrogel preferably has a fibrous structure.

### Advantageous Effects of Invention

The present disclosure can provide a method of preparing a hydrogel whose sterilization and gelling can be achieved by a simple method.

### Brief Description of Drawings

FIG. 1 is a diagram illustrating a synthesis scheme for a chitosan derivative;
FIG. 2 is a graph illustrating the gluconic acid introduction rate in a chitosan derivative;
FIG. 3 is a photograph of medical hydrogels prepared in Examples;
FIG. 4 is a graph illustrating the compressive strength of medical hydrogels prepared in Examples;
FIG. 5 is a photograph of an SEM image of GC54 hydrogel; and
FIG. 6 is a photograph of an SEM image of GC54FM hydrogel.

### Description of Embodiments

A method of preparing a medical hydrogel according to the present embodiment is described below. The medical hydrogel obtained by the preparation method according to the present embodiment is used as a wound dressing that produces a therapeutic effect on wound surfaces of cut wounds, lacerated wounds, contused wounds, burns, bedsores, and the like by maintaining the moist environment and promoting the cell proliferation on the wound surfaces.

The method of preparing a medical hydrogel according to the present embodiment is a method of preparing a medical hydrogel derived from a chitosan derivative containing aldonic acid bound by dehydration condensation to an amino group of a glucosamine unit of chitosan, the method including placing an aqueous solution containing the chitosan derivative in a mold having a predetermined shape, and then subjecting the aqueous solution to high-pressure steam sterilization, to obtain a sterile medical hydrogel having the predetermined shape.

In the present and other embodiments, the chitosan used to synthesize the chitosan derivative is a deacetylated product of chitin. As is well known, the conversion (deacetylation reaction) of chitin to chitosan does not proceed completely, and the sugar chain partially includes N-acetylglucosamine. Commercially available chitosans usually have a deacetylation rate within the range of 50 to 100%, often have a deacetylation rate of about 70 to 90%.

Unless otherwise specified, the chitosan used in relation to the present embodiment means a chitosan having a deacetylation rate of 50 to 100%, and therefore, encompasses chitosans whose deacetylation rates are not 100%.

The aldonic acid used to synthesize the chitosan derivative used in the present embodiment is a general term of a derivative obtained by oxidizing a monosaccharide, the derivative being a carboxylic acid containing a carboxyl group derived by conversion of the formyl group at the 1-position of aldose. The aldonic acid can be represented by, for example, Formula (I) below. In Formula (I), n is an integer of 2 to 4.

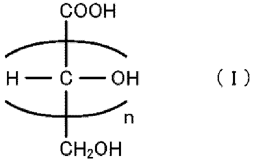

A preferred example of the aldonic acid used to synthesize the chitosan derivative used in the present embodiment is gluconic acid. Threonic acid or xylonic acid can also be preferably used. Further, any of the carboxylic acids that are known as aldonic acids may be used, and examples of such carboxylic acids include galactonic acid, mannonic acid, lyxonic acid, erythronic acid, ribonic acid, arabinonic acid, allonic acid, altronic acid, gulonic acid, idonic acid, and talonic acid.

As can be understood from the above description, the chitosan derivative used in the present embodiment can be generally represented, as shown in Formula (II) below, as a chitosan derivative containing repeat units (repeating units) each including a part where aldonic acid is bound to the amino group of a glucosamine unit and a part where an acetylglucosamine unit is remaining. In Formula (II), n is an integer of 2 to 4.

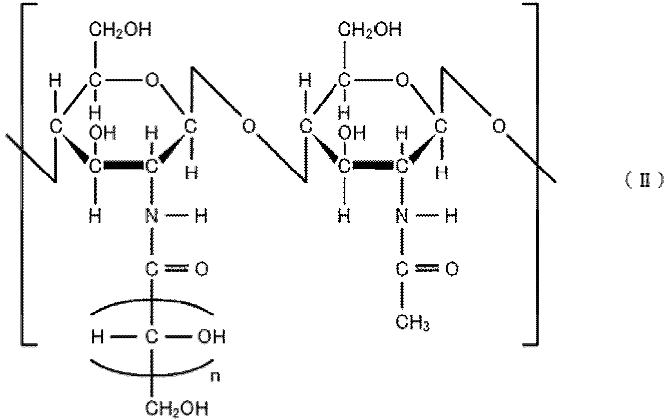

Thus, when the aldonic acid is gluconic acid, the chitosan derivative used in the present embodiment can be generally represented as a chitosan derivative containing the repeat unit shown in Formula (III) below.

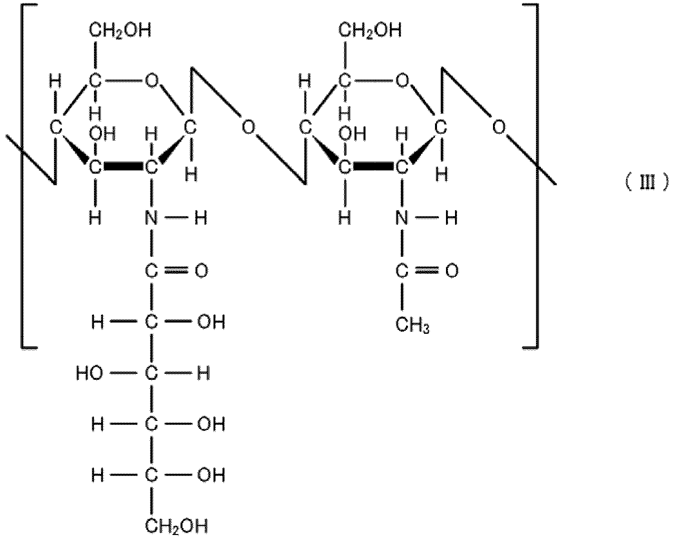

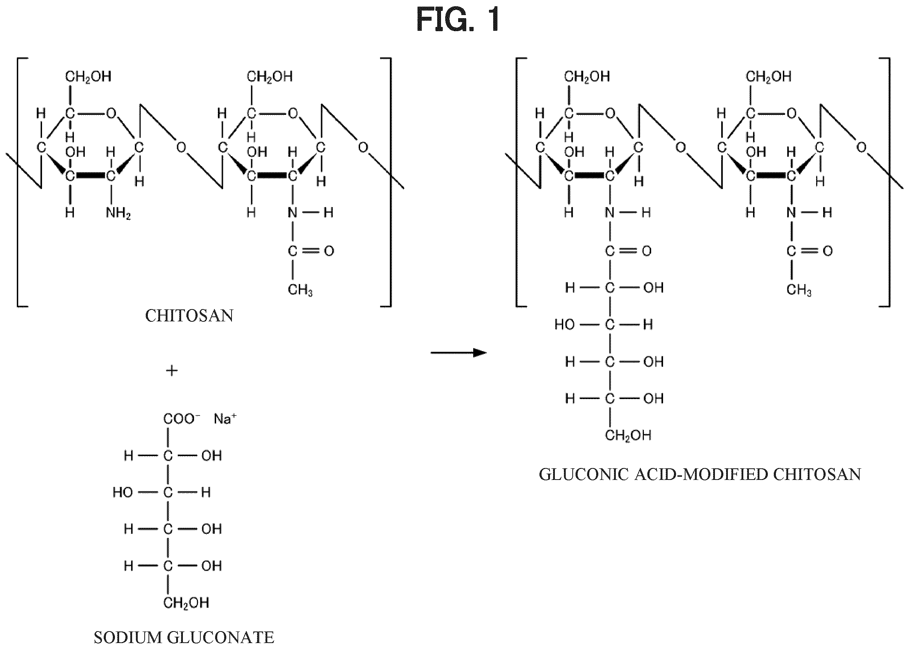

The chitosan derivative used in the present embodiment can be synthesized by subjecting aldonic acid to condensation dehydration reaction with chitosan, to bind (introduce) the aldonic acid to the amino group at the 2-position of glucosamine units of the chitosan. The aldonic acid is generally reacted as an appropriate salt (for example, Na salt). FIG. 1 illustrates a synthesis scheme for a case where gluconic acid is used as the aldonic acid.

The condensation dehydration reaction can be carried out at room temperature under acidic conditions using a condensing agent (dehydration-condensation agent). Preferred examples of the condensing agent in the condensation dehydration reaction between the chitosan and the aldonic acid include, but are not limited to, 1-ethyl-3-(3-dimethylaminopropyl)carbodiimide hydrochloride (EDC), which functions to activate the carboxyl group of the aldonic acid, and N-hydroxysuccinimide (NHS), which functions to suppress side reactions.

The rate of introduction of the aldonic acid such as gluconic acid into the chitosan can be adjusted by changing the amount of the aldonic acid used relative to the chitosan, and changing the amount of the condensing agent used. By increasing the relative amount of the aldonic acid used, and increasing the amount of the condensing agent used, the introduction rate can be increased. The rate of introduction of the aldonic acid in the chitosan derivative used in the present embodiment is, for example, 5 to 60%.

The rate of introduction of the aldonic acid such as gluconic acid (into the chitosan) is represented by Equation (A) below. $Aldonic acid introduction rate \left(%\right) = \left[Y/\left(X+Y\right)\right] \times 100$

In Equation (A), X is the amount of substance (number of moles) of glucosamine units contained in the chitosan-introduced product, and Y is the amount of substance (number of moles) of aldonic acid-introduced glucosamine units contained in the chitosan derivative.

The introduction rate can be calculated by dissolving the chitosan derivative (aldonic acid-modified chitosan) in an appropriate concentration of aqueous hydrochloric acid solution, and then adding an equimolar concentration, with respect to the hydrochloric acid, of aqueous sodium hydroxide solution to the resulting solution while measuring the change in the electrical conductivity.

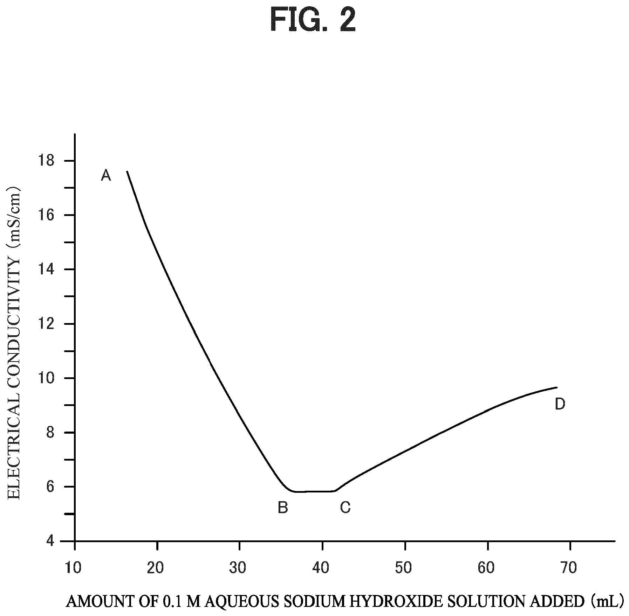

This measurement method is described below in more detail by way of FIG. 2 for the case of gluconic acid-modified chitosan (which may be hereinafter simply referred to as GC), which is obtained using gluconic acid as the aldonic acid. When 0.1 M sodium hydroxide is added to 0.1 M aqueous hydrochloric acid solution containing gluconic acid-modified chitosan dissolved therein, H⁺ in the solution decreases due to neutralization reaction, resulting in a decrease in the electrical conductivity of the aqueous solution (A to B in FIG. 2). Upon the completion of the neutralization reaction, protonated amino groups in the glucosamine units begin to undergo deprotonation, and the electrical conductivity does not change until the completion of the deprotonation (B to C in FIG. 2). Upon the completion of the deprotonation, OH⁻ in the aqueous solution increases, resulting in an increase in the electrical conductivity (C to D in FIG. 2). Here, the amount of substance of the sodium hydroxide added to the aqueous solution in B to C in FIG. 2 corresponds to the amount of substance of the protonated amino groups in the glucosamine units. Taking into account the fact that the amino groups in most glucosamine units are protonated in this aqueous hydrochloric acid solution, the amount of substance of the sodium hydroxide added to the aqueous solution in B to C in FIG. 2 corresponds to the amount of substance of glucosamine units that are not modified with gluconic acid. Therefore, the gluconic acid introduction rate can be determined by calculating the amount of substance of glucosamine units in each of gluconic acid-modified chitosan and gluconic acid-unmodified chitosan by this measurement method, and comparing the calculated amounts of substance.

In the present embodiment, an aqueous solution containing the chitosan derivative synthesized as described above is placed in a mold having a predetermined shape, and subjected to high-pressure steam sterilization. By this, gelling of the aqueous solution containing the chitosan derivative occurs, and at the same time, sterilization can be achieved, resulting in formation of a biocompatible medical hydrogel having a predetermined shape.

The high-pressure steam sterilization can be carried out using an autoclave. The temperature and the time for this treatment are not limited as long as the sterilization is possible. The treatment may be carried out at 2 atm at not less than 121°C for not less than 15 minutes as in common autoclave sterilization, or may also be carried out for 20 minutes to 2 hours.

The aqueous solution containing the chitosan derivative can be obtained by dissolving a dry powder of the above-described chitosan derivative in an aqueous acidic solution such as an aqueous hydrochloric acid solution, and then adding an alkali such as sodium hydroxide thereto to neutralize the resulting solution to a pH that is almost neutral (pH 7). The content of the chitosan derivative in the aqueous solution is not limited as long as the chitosan derivative is sufficiently dissolved in the aqueous solution, and may be, for example, 1 to 5% (weight/volume).

The medical hydrogel formed as described above is an elastic hydrogel. In accordance with the shape of the mold used, the resulting medical hydrogel can have various shapes suitable for its intended use, such as a sheet-like shape or a spherical shape. Further, the medical hydrogel has excellent biocompatibility since, as described above, it is obtained using no cross-linking agent at all, and using no additives harmful to the living body.

Further, by using a chitosan derivative having a higher aldonic acid introduction rate, and by increasing the time of the high-pressure steam sterilization, a medical hydrogel having higher mechanical strength can be obtained. The higher the mechanical strength of the medical hydrogel, the more easily it can be handled when, for example, the medical hydrogel is processed into a size suitable for the size of a wound surface, or is attached to an external material for retaining the medical hydrogel on a wound surface. Thus, a medical hydrogel having a mechanical strength suitable for its intended use can be obtained.

In the present embodiment, the medical hydrogel is produced without freezing and thawing of the aqueous solution containing the chitosan derivative. Therefore, the resulting medical hydrogel has a fibrous and dense internal structure. Chitosan contained in medical hydrogels is degraded by enzymes in the body fluid that exudes from the wound. However, the enzymatic degradation takes a long time in medical hydrogels having a fibrous and dense structure. Therefore, the medical hydrogel according to the present embodiment can remain on the wound surface for a long period of time, thereby accelerating the wound healing.

As described above, in the present embodiment, a medical hydrogel can be obtained by the single step of subjecting the aqueous solution containing the chitosan derivative to high-pressure steam sterilization. Therefore, the embodiment is advantageous in that the medical hydrogel can be prepared by a very simple method. Furthermore, a medical hydrogel suitable for its intended use can be easily prepared in medical institutions and the like where an autoclave is available.

### Examples

### <Synthesis of Gluconic Acid-Modified Chitosan>

After dissolving 2-morpholinoethanesulfonic acid in 300 ml of distilled water at a concentration of 23.5 mM (pH 4.0), 3.0 g of chitosan (trade name, "Chitosan LL"; deacetylation rate, 80%; manufactured by Yaizu Suisankagaku Industry Co., Ltd.) was dissolved in the resulting solution, and 1M aqueous hydrochloric acid solution was added thereto to adjust the pH to 4.0. As shown in Table 1, sodium gluconate (hereinafter simply referred to as GA), 1-ethyl-3-(3-dimethylaminopropyl) carbodiimide hydrochloride (hereinafter simply referred to as EDC), and N-hydroxysuccinimide (hereinafter simply referred to as NHS) were dissolved in this aqueous solution, and the resulting solution was stirred at room temperature for 24 hours, to introduce gluconic acid to the amino group at the 2-position of glucosamine units of chitosan (see FIG. 2).

Subsequently, 1M aqueous sodium hydroxide solution was added to the reaction solution to adjust the pH to 8.0, and then 99.5% ethanol was added thereto to precipitate gluconic acid-modified chitosan and unreacted sodium gluconate, followed by collecting the resulting precipitate by centrifugation. Thereafter, the precipitate was sealed in a dialysis membrane, and immersed in distilled water for 1 week to remove sodium gluconate. In this process, the distilled water was replaced twice a day. Subsequently, the precipitate and the aqueous solution in the dialysis membrane were collected, and 99.5% ethanol was added thereto to precipitate gluconic acid-modified chitosan, followed by collecting the resulting precipitate. Thereafter, freeze-drying was performed to obtain a dry powder of gluconic acid-modified chitosan, which was then stored.

Three kinds of dry powders of gluconic acid-modified chitosan were prepared at different raw material ratios. These gluconic acid-modified chitosans are referred to as GC8, GC26, and GC54, respectively. The gluconic acid introduction rate was then calculated for each of GC8, GC26, and GC54. The gluconic acid introduction rate was calculated as mentioned above by dissolving 0.2 g of the dry powder of gluconic acid-modified chitosan in 40 ml of 0.1 M aqueous hydrochloric acid solution, and adding 0.1 M aqueous sodium hydroxide solution to the resulting aqueous solution while measuring the change in the electrical conductivity. Table 1 shows the preparation conditions and the gluconic acid introduction rate of each gluconic acid-modified chitosan.

**Table 1**

| | **Chitosan (g)** | **GA(g)** | **EDC(g)** | **NHS(g)** | **Gluconic acid introduction rate (%)** |
|---|---|---|---|---|---|
| **GC8** | **10** | **2.69** | **2.37** | **0.74** | **8** |
| **GC26** | **10** | **5.38** | **4.73** | **1.42** | **26** |
| **GC54** | **5** | **10.77** | **9.46** | **2.84** | **54** |

### <Sterilization by Autoclaving and Preparation of Hydrogel>

The dry powder of each gluconic acid-modified chitosan synthesized as described above was added to distilled water (2.0% [weight/volume]). The powder was then completely dissolved by adding 0.1 M aqueous hydrochloric acid solution thereto. Subsequently, 0.1 M or 1.0 M aqueous sodium hydroxide solution was added thereto to adjust the pH to 7.0.

The aqueous gluconic acid-modified chitosan solution was placed in a glass container having a cylindrical shape with an inner diameter of 15 mm, and then the container was sealed. The container was then placed in an autoclave, and high-pressure steam sterilization (121°C, 2 atm) was carried out. The treatment time was set to 20 minutes, 60 minutes, or 120 minutes, respectively.

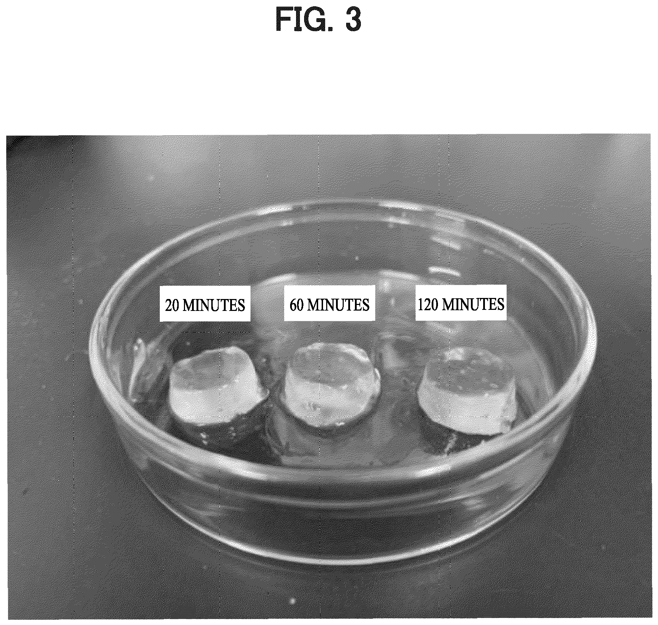

As a result, all aqueous gluconic acid-modified chitosan solutions prepared with any of the treatment times showed gelling to produce medical hydrogels (hereinafter referred to as GC8 hydrogel, GC26 hydrogel, and GC54 hydrogel, respectively). FIG. 3 is a photograph of GC54 hydrogel as a representative.

### <Compression Test>

Each medical hydrogel obtained as described above was subjected to measurement of the compressive strength. Each medical hydrogel was placed in a glass container, and a compression test was carried out by lowering an indenter having a diameter of 10 mm at 5 mm/min from above. The compressive strength was measured at the time when the strain reached 10%.

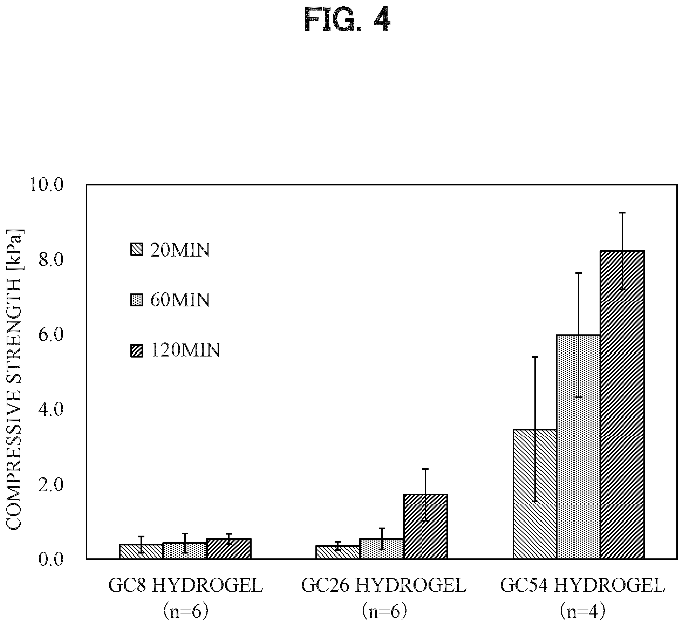

The results are illustrated in FIG. 4. GC8 hydrogel hardly showed differences in the compressive strength among the cases with the different lengths of the treatment time. On the other hand, GC26 hydrogel and GC54 hydrogel tended to show higher compressive strength as the treatment time increased. It was found that medical hydroxy gels having higher mechanical strength can be obtained as the gluconic acid introduction rate increases, and as the treatment time of the high-pressure steam sterilization increases.

### <Comparison of Structure with Hydrogel Prepared by Freezing and Thawing>

An aqueous gluconic acid-modified chitosan solution using the dry powder of GC54 described above was placed in a metal mold, and frozen at 20°C for 12 hours. Thereafter, the frozen product was left to stand at room temperature to thaw it, to produce a medical hydrogel (hereinafter referred to as GC54FM hydrogel).

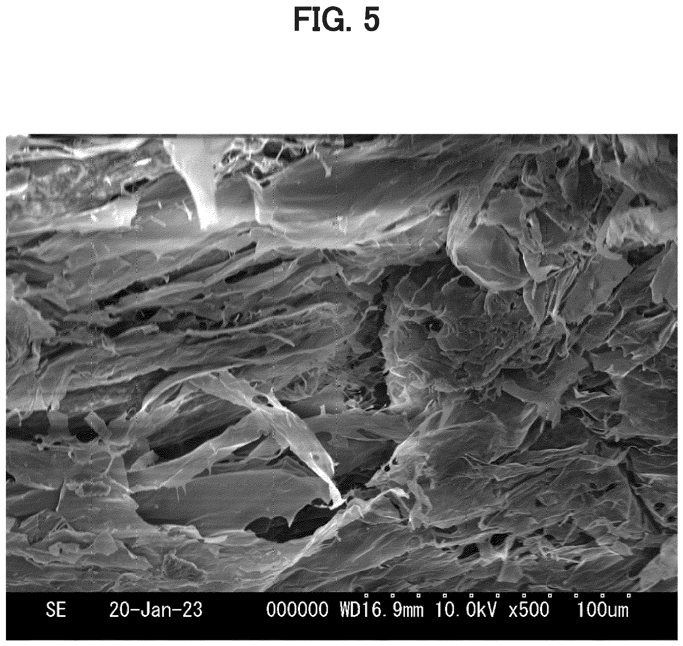

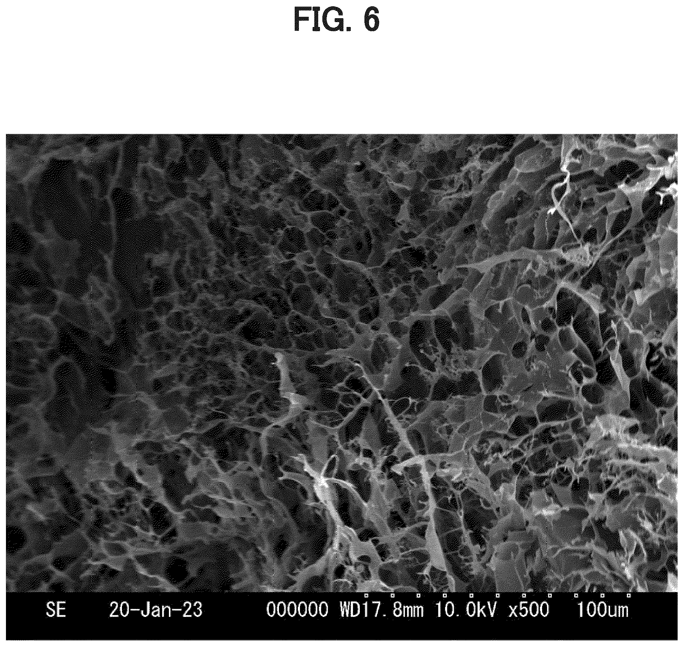

Scanning electron microscope (SEM) images of GC54 hydrogel and GC54FM hydrogel were captured. FIG. 5 is the SEM image of GC54 hydrogel. FIG. 6 is the SEM image of GC54FM hydrogel.

Since freezing and thawing were carried out in the production of the GC54FM hydrogel, the GC54FM hydrogel showed a large number of pores formed due to ice crystals generated during the freezing. On the other hand, since freezing and thawing were not carried out in the production of the GC54 hydrogel, the GC54 hydrogel did not show pores like those in the GC54FM hydrogel, and exhibited a fibrous and dense structure.

This application claims the benefit of Japanese Patent Application No. 2022-30659, filed on March 1, 2022.

### Industrial Applicability

The medical hydrogel can be used as a wound dressing for use in the treatment of wound surfaces of cut wounds, lacerated wounds, contused wounds, burns, bedsores, and the like.

## Claims

1. A method of preparing a medical hydrogel, the medical hydrogel being derived from a chitosan derivative containing aldonic acid bound by dehydration condensation to an amino group of a glucosamine unit of chitosan,
the method comprising placing an aqueous solution containing the chitosan derivative in a mold having a predetermined shape, and then subjecting the aqueous solution to high-pressure steam sterilization, to obtain a sterile medical hydrogel having the predetermined shape.

2. The method of preparing a medical hydrogel according to claim 1, wherein the high-pressure steam sterilization is carried out at not less than 121°C for 15 minutes to 2 hours.

3. The method of preparing a medical hydrogel according to claim 1, wherein the chitosan has a deacetylation rate of 70 to 90%, and the chitosan derivative has an introduction rate of the aldonic acid of 5 to 60%.

4. The method of preparing a medical hydrogel according to claim 1, wherein the aldonic acid is gluconic acid.

5. The method of preparing a medical hydrogel according to claim 1, wherein the resulting medical hydrogel has a fibrous structure.

6. A medical hydrogel obtained by the method according to any one of claims 1 to 4, wherein the medical hydrogel has a fibrous structure.

## Patentansprüche

1. Verfahren zur Herstellung eines medizinischen Hydrogels, wobei das medizinische Hydrogel von einem Chitosan-Derivat abgeleitet ist, das eine Aldonsäure enthält, die durch Dehydratisierungskondensation an eine Aminogruppe einer Glucosamin-Einheit des Chitosans gebunden ist,
wobei das Verfahren das Anordnen einer wässrigen Lösung, die das Chitosan-Derivat enthält, in einer Form mit einer vorbestimmten Gestalt und das anschließende Unterziehen der wässrigen Lösung einer Hochdruckdampfsterilisation umfasst, um ein steriles medizinisches Hydrogel mit der vorbestimmten Gestalt zu erhalten.

2. Verfahren zur Herstellung eines medizinischen Hydrogels nach Anspruch 1, wobei die Hochdruckdampfsterilisation bei nicht weniger als 121 °C für 15 Minuten bis 2 Stunden durchgeführt wird.

3. Verfahren zur Herstellung eines medizinischen Hydrogels nach Anspruch 1, wobei das Chitosan einen Deacetylierungsgrad von 70 bis 90 % aufweist und das Chitosan-Derivat eine Einführungsrate der Aldonsäure von 5 bis 60 % aufweist.

4. Verfahren zur Herstellung eines medizinischen Hydrogels nach Anspruch 1, wobei die Aldonsäure Gluconsäure ist.

5. Verfahren zur Herstellung eines medizinischen Hydrogels nach Anspruch 1, wobei das erhaltene medizinische Hydrogel eine faserige Struktur aufweist.

6. Medizinisches Hydrogel, erhältlich durch das Verfahren nach einem der Ansprüche 1 bis 4, wobei das medizinische Hydrogel eine faserige Struktur aufweist.

## Revendications

1. Procédé de préparation d'un hydrogel médical, l'hydrogel médical étant dérivé d'un dérivé de chitosane contenant de l'acide aldonique lié par condensation par déshydratation à un groupe amino d'une unité glucosamine de chitosane,
le procédé comprenant le placement d'une solution aqueuse contenant le dérivé de chitosane dans un moule ayant une forme prédéterminée, et ensuite la soumission de la solution aqueuse à une stérilisation à la vapeur à haute pression, afin d'obtenir un hydrogel médical stérile ayant la forme prédéterminée.

2. Procédé de préparation d'un hydrogel médical selon la revendication 1, dans lequel la stérilisation à la vapeur à haute pression est effectuée à une température non inférieure à 121 °C pendant 15 minutes à 2 heures.

3. Procédé de préparation d'un hydrogel médical selon la revendication 1, dans lequel le chitosane a un taux de désacétylation de 70 à 90%, et le dérivé de chitosane a un taux d'introduction de l'acide aldonique de 5 à 60 %.

4. Procédé de préparation d'un hydrogel médical selon la revendication 1, dans lequel l'acide aldonique est l'acide gluconique.

5. Procédé de préparation d'un hydrogel médical selon la revendication 1, dans lequel l'hydrogel médical résultant a une structure fibreuse.

6. Hydrogel médical obtenu par le procédé selon l'une quelconque des revendications 1 à 4, dans lequel l'hydrogel médical a une structure fibreuse.
